# FASCICULE DE BREVET EUROPEEN

(11) **EP 0 869 108 B1**
(45) Date de publication et mention de la délivrance du brevet: **05.09.2001**
(21) Numéro de dépôt: 98400736.9
(22) Date de dépôt: 30.03.1998
(51) Int. Cl.: C07C 11/02, C07C 41/06

(54) **Procédé de production d'éther et d'oléfine à partir de coupe hydrocarbonée contenant au moins une oléfine tertiaire par synthèse puis décomposition d'éther comportant une première étape de purification d'oléfine par fractionnement**
Verfahren zur Herstellung von Olefinen und Ether aus einen Kohlenwasserstoffschnitt der wenigstens ein tertiäres Olefin enthält, durch Synthese und nachfolgende Spaltung, das eine Fraktionnierung als erste Stufe zur Reinigung der Olefine umfasst
Process for the preparation of an ether and an olefin from a hydrocarbon fraction containing at least one tertiary olefin by synthesis followed by composition of the ether, comprising a fractionnation as a first olefin purification step

(30) Priorité: 02.04.1997 FR 9704118
(43) Date de publication de la demande: 07.10.1998
(73) Titulaire: INSTITUT FRANCAIS DU PETROLE, 92852 Rueil-Malmaison Cedex (FR)
(72) Inventeur: Marion, Marie-Claire, 69100 Villeurbanne (FR); Coupard, Vincent, 69002 Lyon (FR); Forestiere, Alain, 69390 Vernaison (FR); Travers, Philippe, 92500 Rueil Malmaison (FR); Viltard, Jean-Charles, 26000 Valence (FR)
(74) Mandataire: Andréeff, François

(56) Documents cités:
- FR-A- 2 527 201
- US-A- 4 447 668
- US-A- 5 567 860

## Description

L'invention concerne un procédé intégré de synthèse d'oléfines tertiaires pures, à partir de coupes hydrocarbonées en contenant, comprenant une étape d'éthérification de ladite coupe hydrocarbonée par au moins un alcool dans laquelle on forme un produit comprenant au moins un éther alkylique tertiaire que l'on envoie ensuite dans une zone de décomposition d'éther(s) alkylique(s) tertiaire(s) à partir de laquelle on récupère un produit comprenant au moins une oléfine tertiaire de haute pureté. Elle concerne en particulier un procédé de production d'isobutène de haute pureté, à partir d'une coupe C₄ hydrocarbonée en contenant, comprenant la formation, à partir de cette coupe et de méthanol, d'éther méthylique de l'alcool tertiobutylique (MTBE initiales anglaises de Methyl-Tertio-Butyl-Ether), puis la décomposition du MTBE et le fractionnement du produit de décomposition en alcool méthylique et en isobutène purifié. Le procédé selon la présente invention s'applique également à la synthèse de toute oléfine tertiaire à partir d'éther alkylique tertiaire [par exemple ETBE (éther éthylique de l'alcool tertiobutylique des initiales anglaises Ethyl-Tertio-Butyl-Ether), ETAE (éther éthylique de l'alcool tertioamylique des initiales anglaises Ethyl-Tertio-Amyl-Ether), TAME (éther méthylique de l'alcool tertioamylique des initiales anglaises Tertio-Amyl-Methyl-Ether), isopropyl-tertio-butyl-éther]. La suite de la description de la présente invention, et tout particulièrement les conditions opératoires, sont données à titre indicatif pour la synthèse d'isobutène à partir de coupes hydrocarbonées contenant essentiellement des hydrocarbures à 4 atomes de carbone (dont l'isobutène) par synthèse et décomposition du MTBE.

Il existe diverses voies de production d'isobutène de haute pureté exploitées industriellement. La plus ancienne est le procédé par extraction à l'acide sulfurique, mais il est cher et obsolète ; il est réputé polluant car il produit des rejets d'acide usé. De plus, le rendement en isobutène ne dépasse pas 90 %. La firme ARCO utilise la voie par déshydratation de l'alcool tertio-butylique (ABT), ce dernier étant un sous-produit obtenu avec leur procédé de production d'oxyde de propylène. Le procédé par déshydrogénation de l'isobutane s'est développé au cours de ces dernières années par suite de la demande importante et croissante en MTBE. Toutefois, ce procédé n'est mis en oeuvre de façon rentable que pour de très grosses capacités de production.

La production d'isobutène de haute pureté par craquage du MTBE est aussi bien adaptée pour des petites capacités que pour des grosses capacités. De plus, cette voie bénéficie de toute l'infrastructure relative à l'importance croissante des éthers dans les essences reformulées. De nombreuses raffineries, partout dans le monde, possèdent des installations de productions de MTBE par exemple. D'autre part il y a également un marché d'échange du MTBE au niveau mondial. Cela signifie que la production d'isobutène de haute pureté via la formation et la décomposition du MTBE, peut être mise en oeuvre aisément partout dans le monde, y compris en dehors des raffineries.

L'idée de produire de l'isobutène pur par décomposition d'éther, et plus particulièrement du MTBE, est connue depuis longtemps, mais les procédés de mise en oeuvre proposés par l'art antérieur présentent certains inconvénients.

Ainsi dans le procédé développé par SUMITOMO, décrit par exemple dans la demande de brevet EP-A-68 785, la réaction de décomposition du MTBE est réalisée en phase liquide, en présence d'un catalyseur solide acide de type résine échangeuse d'ions. Deux flux de produit sont obtenus : l'isobutène et le méthanol. Tel que le schéma est décrit, l'isobutène est directement obtenu en tête d'une colonne à distiller sans autre étape de purification. L'isobutène ainsi obtenu contient un certain nombre d'impuretés à commencer par une petite fraction de méthanol qui est distillée par azéotropie, du diméthyléther (DME) composé volatil formé par condensation du méthanol en présence d'un catalyseur acide. Il est probable que la pureté de l'isobutène soit alors insuffisante pour une utilisation telle que la fabrication de polyisobutène ou d'autres copolymères. En outre, aucun moyen ne permet apparemment d'éviter l'accumulation d'impuretés lourdes telles que les dimères de l'isobutène ou l'éther méthylique de l'alcool butylique secondaire (MSBE), ce qui à terme se traduit fatalement par une baisse de pureté des produits.

Dans le procédé développé par ERDOLCHEMIE, décrit par exemple dans le brevet US-A-4 409 421, la purification de l'isobutène formé comprend une élimination de l'alcool résiduel entraîné avec l'oléfine tertiaire par adsorption. Cette méthode présente l'inconvénient d'avoir à régénérer régulièrement l'adsorbant. En outre la récupération de la majeure partie de l'alcool issu de la décomposition n'est pas solutionnée.

Plus récemment, la même société décrit, dans le brevet US-A-5 095 164, la mise en oeuvre de la réaction de décomposition dans un appareillage de distillation. Le catalyseur est alors placé dans le fond de la colonne au niveau du rebouilleur. Cette mise en oeuvre particulière est limitative sur le plan de la température réactionnelle, directement imposée par la nature de l'éther et la pression opératoire. En outre, elle favorise vraisemblablement la formation de sous-produits de réaction tels que la formation de dimères de l'isobutène et/ou la formation de diméthyléther. A ce propos, la qualité et/ou le devenir des produits ne sont pas clairement explicités.

Par ailleurs, la société BASF décrit, dans le brevet US-A-4 287 379, un schéma intégrant à la fois l'étape de synthèse de l'éther, sa séparation, puis l'étape de décomposition de l'éther pour produire l'isobutène. Toutefois, pour éviter certaines étapes de purification, l'éthérification est faite avec un alcool en C3 ou C4, ce qui est un inconvénient majeur face au marché international du MTBE.

Cette société a également décrit dans le texte du brevet US-A-4 320 232 un procédé de préparation conjointe de MTBE et d'isobutène comprenant la formation d'éther à partir d'une coupe C4 contenant de l'isobutène par réaction de cette coupe sur un mélange d'alcool contenant du méthanol et des alcools en C3 ou C4 au cours de laquelle on forme un mélange contenant du MTBE et des éthers alkyliques tertiaires des alcools en C3 et C4 que l'on sépare en une fraction contenant le MTBE et en une fraction contenant ces éthers alkyliques tertiaires des alcools en C3 et C4 qui est envoyée dans une zone de décomposition dans laquelle on forme de l'isobutène. L'emploi d'un mélange d'alcool complique singulièrement le procédé en particulier au niveau de la séparation et de la purification de produits. Par ailleurs ce procédé implique le plus souvent l'utilisation de deux étapes d'éthérification et d'une distillation intermédiaire entre ces deux étapes.

Enfin, nous pouvons encore citer les deux schémas du procédé SNAMPROGETTI présentés dans Chemical Economy & Engineering Review, vol. 14 n° 6 Juin 1982, incluant à la fois l'étape de synthèse du MTBE et l'étape de décomposition du MTBE pour la production d'isobutène. Il apparaît dans ces schémas qu'une certaine perte d'eau par entraînement et/ou saturation du flux isobutène, au niveau du lavage de l'isobutène pour éliminer l'alcool, n'est pas prise en compte. Cela peut se traduire à terme soit par une baisse du débit d'eau de lavage, soit par une perte d'efficacité de cette section de lavage. Cela peut nuire alors à la qualité de l'isobutène produit. Par ailleurs selon ces schémas la fraction hydrocarbonée issue de la colonne d'extraction à l'eau qui contient une quantité relativement importante d'eau libre est envoyée en totalité dans la colonne de fractionnement permettant de récupérer l'isobutène purifié ce qui implique que cette colonne doit traiter une quantité de produit importante et donc avoir des caractéristiques de dimensionnement importante ce qui rend le procédé particulièrement onéreux et délicat à mettre en oeuvre.

Le procédé de production d'isobutène décrit dans le brevet US 4,447,668 prévoit, en aval de l'étape de décomposition de l'éther, l'introduction, dans une zone de lavage à l'eau de la totalité de l'effluent issue de l'étape de décomposition. Dans un tel schéma, il est inévitable qu'une partie de l'éther soit entraînée avec la fraction aqueuse récupérée en fond de la colonne de lavage, d'où une perte substantielle de l'éther non décomposé, qui ne peut pas être recyclé. Il est également inévitable que de l'eau de lavage soit entraînée avec la fraction organique, récupéré en tête de la colonne d'extraction par lavage, ce qui nécessite une colonne de fractionnement de l'isobutène présentant des caractéristiques de dimensionnement importante. Il en est de même de la colonne d'extraction à l'eau qui reçoit la totalité de l'effluent de la zone de décomposition.

Le procédé selon l'invention permet de remédier aux inconvénients précités. Il concerne un procédé de production d'oléfine(s) tertiaire(s) caractérisée(s) par une (très) haute pureté, à partir d'une coupe hydrocarbonée en contenant, par une méthode comprenant la formation puis la décomposition d'au moins un éther alkylique tertiaire. Ce procédé est donc également un procédé adapté à la valorisation des oléfines tertiaires contenues dans les coupes hydrocarbonées sous forme d'oléfines tertiaires purifiées, utilisables en particulier dans la formation de polymères et/ou de copolymères et/ou sous forme d'éthers alkyliques tertiaires, utilisables en particulier comme additifs dans les carburants pour moteurs à combustion.

L'invention concerne un procédé comprenant la formation puis la décomposition d'éther alkylique tertiaire tel que défini précédemment, en particulier de MTBE ou ETBE, pour la production d'oléfine(s) tertiaire(s), d'isobutène en particulier, de haute pureté. Dans le cas de la décomposition d'autres éthers, on peut obtenir un mélange contenant une pluralité d'oléfines tertiaires. Ainsi, dans le cas de la décomposition du TAME, on obtient un mélange contenant du méthyl-2 butène-1 et du méthyl-2 butène-2.

Outre les zones de réaction proprement dites, le procédé selon l'invention comprend des zones de purification ou récupération ou recyclage des divers produits de façon à optimiser la valorisation des produits mis en oeuvre et minimiser les pertes.

La présente invention concerne un procédé de production d'oléfine tertiaire pure et/ou d'éther alkylique tertiaire à partir de coupe hydrocarbonée contenant au moins une oléfine tertiaire éthérifiable, ledit procédé comprenant :
a) une étape de formation d'au moins un éther alkylique tertiaire par mise en contact dans une zone réactionnelle, comprenant généralement au moins un réacteur (R1), et contenant un catalyseur d'éthérification, d'au moins une coupe hydrocarbonée contenant au moins une oléfine tertiaire éthérifiable avec au moins un alcool généralement primaire ou secondaire, de préférence primaire, ayant généralement de 1 à 6, de préférence de 1 à 4 atomes de carbone par molécule, de préférence l'alcool méthylique ou l'alcool éthylique ou l'isopropanol, de façon encore plus préférée l'alcool méthylique ou l'alcool éthylique,
b) une étape de séparation de la majeure partie du produit issu de l'étape a) en une fraction organique (O1) appauvrie en éther alkylique tertiaire, et de préférence ne contenant pratiquement pas d'éther alkylique tertiaire, et en une fraction organique (E1), enrichie en éther alkylique tertiaire, et de préférence contenant pratiquement la totalité de l'éther alkylique tertiaire formé au cours de l'étape a),
c) une étape de décomposition d'au moins une partie de l'éther alkylique tertiaire contenu dans la fraction organique (E1) de l'étape b), dans une zone réactionnelle comprenant généralement au moins un réacteur (R2) contenant un catalyseur de décomposition dudit éther, en un produit (P1) contenant au moins un alcool et au moins une oléfine tertiaire,
d) une étape de fractionnement d'au moins une partie du produit (P1), et éventuellement de la totalité dudit produit, dans une zone de fractionnement (C1) permettant d'obtenir d'une part une fraction (A) contenant la majeure partie de l'oléfine tertiaire et éventuellement une fraction mineure d'alcool et des composés légers éventuels, contenus initialement dans ladite partie de produit (P1), et d'autre part une fraction (B) contenant la majeure partie de l'alcool formé dans l'étape c) et éventuellement de l'éther non décomposé dans l'étape c),
e) une étape de purification d'au moins une partie de la fraction (A) dans laquelle ladite partie est envoyée dans une zone (L1) d'extraction par lavage à l'eau à partir de laquelle on obtient une fraction aqueuse (C) contenant la majeure partie de l'alcool initialement présent dans ladite partie et une fraction (D) contenant la majeure partie de l'oléfine tertiaire initialement présente dans ladite partie, ladite fraction (D) contenant ladite oléfine tertiaire, de l'eau, éventuellement des composés légers et étant sensiblement exempte d'alcool,

ledit procédé étant caractérisé en ce qu'il comporte une étape f) dans laquelle au moins une partie de la fraction (D) est envoyée dans une zone de séparation (Co) à partir de laquelle on récupère une fraction liquide aqueuse (Le) et une fraction organique liquide (Ohp1) contenant la majeure partie de l'oléfine tertiaire initialement présente dans ladite partie de fraction (D), ladite fraction (Ohp1) contenant ladite oléfine tertiaire, une faible quantité d'eau et éventuellement des composés légers.

Dans une forme particulière de réalisation du procédé selon l'invention, une partie de la fraction organique (E1) contenant de l'éther alkylique tertiaire est envoyée dans des fractions carburant moteur et l'autre partie est envoyée à l'étape c) de décomposition de l'éther alkylique tertiaire.

Selon une autre forme particulière de réalisation, le procédé selon l'invention comprend une étape b1) dans laquelle au moins une partie de la fraction organique (E1) issue de l'étape b) contenant de l'éther alkylique tertiaire est envoyée dans une zone de purification (C4), par exemple par distillation, à partir de laquelle on obtient une fraction lourde (L1) appauvrie en éther alkylique tertiaire contenant par exemple des oligomères et du MSBE (éther méthylique de l'alcool butylique secondaire), qui peut être au moins en partie envoyée à la torche et/ou au moins en partie aux fractions carburant moteur, et une fraction plus légère (E2) enrichie en éther alkylique tertiaire que l'on envoie en majeure partie à l'étape c) de décomposition dudit éther. Selon cette forme de mise en oeuvre, une autre partie de la fraction organique (E1) contenant de l'éther alkylique tertiaire peut être envoyée directement aux fractions carburant moteur et encore une autre partie peut être envoyée directement à l'étape c). Il est également possible d'envoyer la totalité de la fraction organique (E1) contenant de l'éther alkylique tertiaire dans la zone (C4) de l'étape b1). Il est également possible d'envoyer seulement une partie de la fraction organique (E1) à l'étape b1) et tout le reste directement à l'étape c).

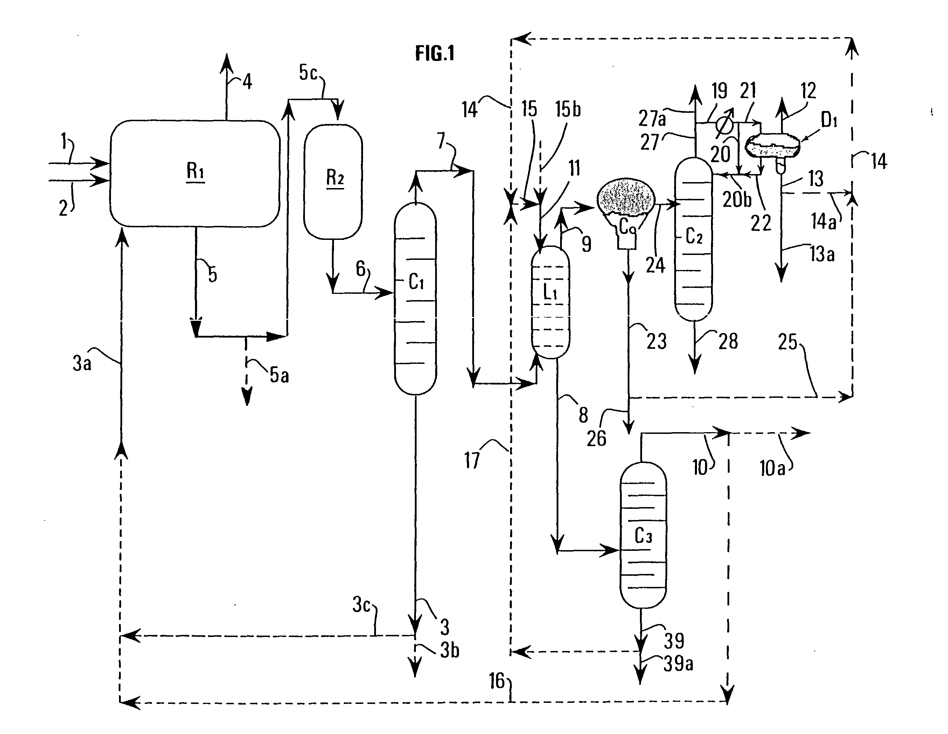

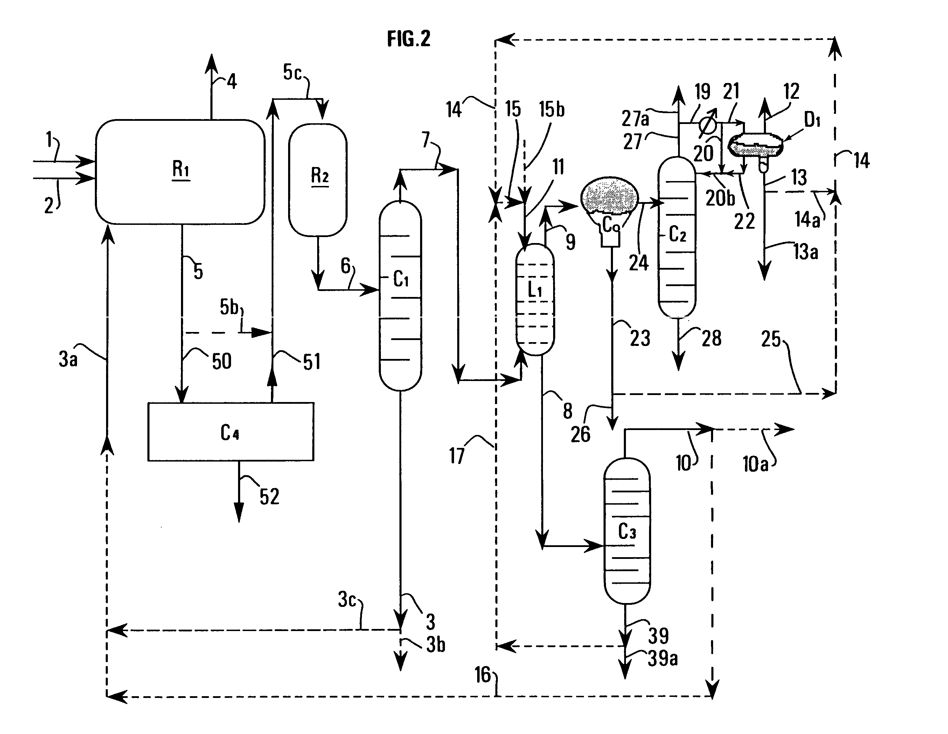

Dans une forme particulière de réalisation du procédé selon l'invention, permettant généralement d'obtenir une oléfine tertiaire de pureté élevée, au moins une partie de la fraction liquide (Ohp1) récupérée à l'étape f) est envoyée dans une étape g) dans une zone de fractionnement (C2) dans laquelle ladite partie de la fraction liquide (Ohp1) est fractionnée d'une part en une fraction (Ohp2) contenant l'oléfine tertiaire et d'autre part en une fraction (F) contenant la majeure partie des composés légers éventuels et éventuellement une faible quantité d'eau résiduelle. La fraction (F) peut être scindée en une fraction gazeuse que l'on évacue par exemple à la torche et en une fraction liquide qui est au moins en partie renvoyée dans la zone de fractionnement (C2) de l'étape g) (ligne (20b) issue de la ligne (20) ou de la ligne (22) sur les figures 1 et 2).

Selon cette forme particulière, il est habituellement préférable que la zone de fractionnement de l'étape g) comporte au moins un moyen permettant de récupérer à partir de la fraction (F) une fraction légère sensiblement anhydre. Le plus souvent ce moyen permet de scinder au moins une partie de la fraction (F) en une fraction légère sensiblement anhydre et en une fraction aqueuse. Ce moyen est par exemple un ballon séparateur muni d'au moins un moyen, par exemple une botte, permettant la décantation et le soutirage d'une fraction aqueuse. Dans ce cas, au moins une partie de la fraction aqueuse obtenue à l'étape g) est de préférence recyclée à l'étape e) dans la zone (L1) d'extraction par lavage à l'eau. Alors, la fraction légère sensiblement anhydre est habituellement scindée en une fraction gazeuse que l'on évacue par exemple à la torche et en une fraction liquide généralement sensiblement anhydre qui est au moins en partie renvoyée dans la zone de fractionnement (C2) de l'étape g). Selon un autre mode de mise en oeuvre, la fraction (F) (ou la fraction légère obtenue à partir de ladite fraction (F)), qui est issue de l'étape g), est au moins en partie envoyée dans une zone de craquage catalytique. Selon une autre variante, la fraction (F) (ou la fraction légère obtenue à partir de ladite fraction (F)) qui est issue de l'étape g) est au moins en partie envoyée dans une zone réactionnelle de synthèse d'éther, de préférence la zone réactionnelle de synthèse d'éther de l'étape a). Selon encore une autre variante, la fraction (F) (ou la fraction légère obtenue à partir de ladite fraction (F)), qui est issue de l'étape g), est au moins en partie envoyée à la torche.

Le plus souvent le procédé de la présente invention comprend une étape h) dans laquelle au moins une partie de la fraction aqueuse (C) issue de l'étape e) est envoyée dans une zone de fractionnement (C3) à partir de laquelle on récupère une fraction (G) contenant la majeure partie de l'alcool initialement présent dans ladite partie et une fraction aqueuse (H) débarrassée de la majeure partie de l'alcool initialement présent dans ladite partie. Selon ce mode de fonctionnement au moins une partie de ladite fraction (G) peut être envoyée dans une zone de synthèse d'éther, de préférence la zone réactionnelle de synthèse d'éther de l'étape a). Il est également possible d'envoyer la totalité de cet alcool dans ladite zone de synthèse d'éther. On peut également récupérer en partie ou en totalité cet alcool pour d'autres usages. Selon ce mode de fonctionnement, au moins une partie de la fraction aqueuse (H) obtenue à l'étape h) peut aussi être au moins en partie recyclée à l'étape e) dans la zone (L1) d'extraction par lavage à l'eau. Selon ce mode de fonctionnement, au moins une partie de la fraction aqueuse (H) obtenue à l'étape f) peut aussi être au moins en partie envoyée dans une zone de traitement des eaux.

Dans une forme préférée de mise en oeuvre du procédé de la présente invention, au moins une partie de la fraction (B) obtenue à l'étape d), contenant la majeure partie de l'alcool formé dans l'étape c) et éventuellement de l'éther non décomposé dans l'étape c), est envoyée dans une zone de synthèse d'éther, de préférence dans la zone de synthèse d'éther de l'étape a). Il est également possible d'envoyer la totalité de cette fraction dans ladite zone de synthèse d'éther. On peut également récupérer en partie ou en totalité cette fraction pour d'autres usages.

Dans une forme préférée de mise en oeuvre du procédé de la présente invention, au moins une partie de la fraction aqueuse (Le) obtenue à l'étape f) est recyclée à l'étape e) dans la zone d'extraction (L1) par lavage à l'eau.

Ces divers recyclages d'eau sont indépendants les uns des autres et peuvent être réalisés ensembles ou séparément. L'eau qui n'est pas recyclée est généralement purgée puis habituellement envoyée vers une zone de traitement des eaux usées. Cette purge est le plus souvent présente au moins sur la fraction aqueuse (H) obtenue à l'étape h) lors de la présence d'une zone (C3). Cette purge permet en particulier d'éviter une éventuelle accumulation de composés dits lourds, par exemple d'alcools lourds.

Les conditions de mise en oeuvre de l'étape a) de la présente invention sont des conditions classiques bien connues de l'homme du métier pour la synthèse d'éther alkylique tertiaire à partir de coupe hydrocarbonée contenant au moins une oléfine tertiaire éthérifiable par au moins un alcool, en particulier un alcool ayant de 1 à 6 atomes de carbone par molécule et le plus souvent le méthanol ou l'éthanol. Les coupes hydrocarbonées que l'on utilise dans le cadre de la présente invention contiennent au moins une oléfine tertiaire et en général d'autres composés hydrocarbonés saturés ou insaturés tels que par exemple d'autres oléfines, des paraffines, éventuellement une faible proportion d'eau, et/ou des oxydes de carbone. Les oléfines tertiaires purifiées que l'on envisage de préparer selon la présente invention sont des composés dont un atome de carbone de la liaison oléfinique est branché. Ces composés ont habituellement de 4 à 10 atomes de carbone par molécule, de préférence de 4 à 8 atomes de carbone par molécule et le plus souvent de 4 à 6 atomes de carbone par molécule. On peut ainsi citer l'isobutène, le méthyl-2 butène-1, le méthyl-2 butène-2, les héxènes tertiaires, les octènes tertiaires et les décènes tertiaires. On peut citer les coupes C₄ et/ou C₅ issues du raffinage ou de la pétrochimie, telles que les coupes C₄ et/ou C₅ de vapocraquage, habituellement après extraction des diènes, les coupes C₄ et/ou C₅ de craquage catalytique, les coupes issues d'isomérisation (hydroisomérisation ou isomérisation squelettales) et les coupes obtenues par déshydrogénation de paraffines. La synthèse d'éther est le plus souvent effectuée en présence d'un catalyseur acide et le plus souvent d'un catalyseur acide solide choisi dans le groupe formé par les résines acides organiques (par exemple des résines sulfoniques) et les résines acides minérales généralement solides dans les conditions de la réaction de synthèse d'éther (par exemple les solides minéraux greffés comportant au moins un groupe organique sulfonique par exemple alkyl-sulfonique, aryl-sulfonique, alkylaryl-sulfonique et en particulier les polysiloxanes greffés et plus particulièrement ceux greffés par au moins un groupe alkyl-sulfonique). Ledit catalyseur peut être une résine commerciale telle que la résine Amberlyst 15 ou 35 ou la résine M 31 de la société DOW-CHEMICAL, ou un polysiloxane greffé commercial. Dans cette étape a) la quantité d'alcool employée est habituellement telle que le rapport molaire alcool/oléfine tertiaire présente dans la charge est d'environ 0,5 : 1 à environ 8 : 1, souvent d'environ 0,8 : 1 à environ 5 : 1, le plus souvent d'environ 0,9 : 1 environ 4 : 1. La température de réaction est habituellement d'environ 20 °C à environ 120 °C, souvent d'environ 30 °C à environ 100 °C, le plus souvent d'environ 40°C à environ 90 °C. La VVH (vitesse volumique horaire) en volume de charge par volume de catalyseur et par heure est habituellement d'environ 0,005 à environ 100, souvent d'environ 0,01 à environ 50 et le plus souvent d'environ 0,1 à environ 10. La pression est habituellement choisie de manière à ce que les constituants présents dans la zone de réaction soient à l'état liquide. Habituellement la pression absolue dans cette zone d'éthérification est d'environ 1 bar à environ 40 bar, souvent d'environ 1 bar à environ 25 bar (1 bar est égal à 0,1 MPa).

Les conditions de mise en oeuvre de l'étape b) de séparation du produit issu de l'étape a) en une fraction organique (O1) ne contenant pratiquement pas d'éther alkylique tertiaire et une fraction organique (E1) enrichie en éther alkylique tertiaire, contenant de préférence pratiquement la totalité de l'éther alkylique tertiaire formé au cours de l'étape a), sont des conditions classiques qui dépendent des composés présents dans le produit issu de l'étape a). Cette séparation peut être effectuée dans des conditions plus ou moins sévères permettant d'obtenir une fraction (O1) contenant encore une faible proportion d'éther. L'homme du métier est à même de choisir les conditions opératoires en vue d'obtenir la séparation souhaitée. Les conditions sont le plus souvent choisies de manière à obtenir une fraction (E1) contenant la quasi totalité de l'éther formé au cours de l'étape a). Dans le cadre de l'invention la zone réactionnelle d'éthérification de l'étape a) peut être distincte de la zone de séparation ou fractionnement de l'étape b), ou bien on peut employer un appareillage comprenant une zone mixte de réaction et de fractionnement (colonne de distillation catalytique) comme cela a par exemple été décrit dans de nombreux brevets et autres publications de l'art antérieur. Dans le cas d'un procédé dans lequel on fabrique dans l'étape a) du MTBE, la colonne de séparation par distillation travaille habituellement sous une pression absolue d'environ 1 à environ 30 bar, identique ou différente de celle régnant dans la zone d'éthérification. Cette colonne comporte habituellement de 3 à 80 plateaux théoriques et souvent de 10 à 50 plateaux théoriques.

Les conditions de mise en oeuvre de l'étape c) de la présente invention sont des conditions classiques de décomposition d'éther alkylique tertiaire bien connues de l'homme du métier. Dans une forme préférée de réalisation cette étape c) sera mise en oeuvre sans addition d'eau supplémentaire au produit introduit dans la zone de décomposition. Il serait cependant possible d'ajouter une certaine quantité d'eau par exemple jusqu'à la limite de solubilité de l'eau dans l'éther que l'on souhaite décomposer. Habituellement les conditions de mise en oeuvre de cette étape c) sont choisies de manière à ce que la majeure partie de l'éther alkylique tertiaire se décompose pour donner un alcool et une oléfine tertiaire. Dans cette zone de décomposition la pression absolue est habituellement d'environ 1 à environ 30 bar, de préférence d'environ 1 à environ 12 bar, la température est habituellement comprise entre 50 °C et 300 °C et de préférence entre 100 °C et 250 °C, la VVH (vitesse spatiale horaire) est habituellement comprise entre 0,1 et 200 h⁻¹ et le plus souvent entre 0,5 et 100 h⁻¹. Dans cette zone on peut utiliser tous les catalyseurs acides bien connus de l'homme du métier. On préfère habituellement employer des catalyseurs solides acides. Ainsi le catalyseur peut être choisi dans le groupe formé par les résines acides organiques et les résines acides minérales généralement solides dans les conditions de la réaction de décomposition dudit éther. Parmi ces composés on emploie le plus souvent ceux choisis dans le groupe formé par les solides minéraux greffés comportant au moins un groupe organique de type alkyl-sulfonique, aryl-sulfonique ou alkylaryl-sulfonique. L'une des formes préférée de mise en oeuvre de cette étape c) utilise un catalyseur choisi dans le groupe formé par les polysiloxanes greffés par au moins un groupe alkyl-sulfonique. Les conditions générales de mise en oeuvre de l'étape d) de fractionnement du produit (P1) issu de l'étape c) de décomposition de l'éther est une étape dont les conditions sont choisies en particulier en fonction des caractéristiques de l'alcool et de l'oléfine tertiaire formés. L'homme du métier est à même de choisir ces conditions pour obtenir la séparation souhaitée entre une fraction contenant la majeure partie de l'alcool et une fraction contenant la majeure partie de l'oléfine. Ainsi par exemple dans le cas de la décomposition du MTBE et de la formation de méthanol et d'isobutène la pression absolue dans la colonne de distillation est d'environ 1 à environ 15 bar, de préférence d'environ 1 à environ 10 bar, identique ou différente de celle régnant dans la zone de décomposition. La température de fond de la colonne dépend à la fois de la pression régnant dans ladite colonne et de la composition du produit de fond, en particulier du rapport molaire entre le méthanol et le MTBE éventuellement présent par suite d'une décomposition partielle de cet éther dans l'étape c). Dans le cas d'une unité traitant 1 kg/h de MTBE la colonne de distillation comporte habituellement entre 3 et 80 plateaux théoriques et le plus souvent entre 10 et 50 plateaux théoriques.

Dans l'étape e) de purification au moins une partie de la fraction (A) contenant la majeure partie de l'oléfine tertiaire obtenue à l'étape c) est envoyée dans une zone d'extraction (L1) par lavage à l'eau. La quantité d'eau utilisée pour ce lavage est habituellement telle que le rapport volumique entre le volume de ladite quantité d'eau introduite dans ladite zone d'extraction et celui de ladite partie de fraction (A) introduite dans ladite zone d'extraction (Vₑₐᵤ/V_{A}) soit d'environ 0,005 à environ 20. Le plus souvent cette quantité d'eau est telle que le rapport Vₑₐᵤ/V_{A} soit d'environ 0,005 à environ 10, de préférence d'environ 0,01 à environ 5 et de manière encore plus préférée d'environ 0,02 à environ 1. Le débit d'eau dans cette zone de lavage (L1) est très souvent régulé en fonction du maintien d'un niveau de fond dans la zone de fractionnement (C3) de l'eau et de l'alcool, dans le cas de la présence d'une telle zone (C3). Ce niveau de fond peut être défini comme le niveau minimal nécessaire au bon fonctionnement de ladite zone. Ce paramètre est un paramètre classique bien connu de l'homme du métier. Cette régulation est souvent faite en mode manuel par les opérateurs, mais il est possible que cette régulation soit effectuée par une boucle de régulation automatique dite LCR des initiales anglo-saxonnes Level Control Regulation. Quel que soit le mode de régulation choisi, la quantité d'eau peut être généralement ajustée à l'aide d'un moyen d'introduction d'eau d'appoint dans la zone (L1). Cet appoint d'eau permet en particulier de compenser les pertes d'eau dues à l'entraînement d'eau et/ou à la saturation du flux hydrocarboné traité, ainsi que le remplacement de l'eau éventuellement purgée. Cette zone d'extraction (L1) est habituellement une colonne à plateaux qui opère à une température d'environ 1 à environ 100 °C, de préférence d'environ 10 à environ 60 °C. La pression absolue dans cette zone est d'environ 1 à environ 20 bar, le plus souvent d'environ 1 à environ 15 bar, identique ou différente de celle régnant dans la zone de fractionnement de l'étape d).

L'étape f) qui comprend une zone de séparation d'au moins une partie de la fraction (D) issue de la zone (L1) de l'étape e) en une fraction liquide aqueuse (Le) et en une fraction liquide hydrocarbonée (Ohp1), dans une zone (Co), est une étape classique bien connue de l'homme du métier. Cette étape est habituellement mise en oeuvre dans un appareil dénommé coalesceur, dans lequel l'eau se rassemble à la partie inférieure de l'appareil par coalescence. Les conditions de température et de pression régnant dans cette zone sont dans les mêmes gammes que celles régnant dans la zone (L1). La pression (respectivement la température) peut être identique ou différente de celle régnant dans ladite zone (L1). Dans la zone (Co), on sépare ainsi l'eau libre contenue dans le produit (D) issu de l'étape e). De plus, ladite zone (Co) a également le plus souvent une fonction de zone ou ballon de charge pour la zone de purification (C2) de l'oléfine tertiaire, dans le cas de la présence d'une telle zone (C2). Tout autre moyen connu de l'homme du métier peut être employé dans le cadre de la présente invention. A titre d'exemple, on peut citer l'utilisation d'un absorbant ayant une sélectivité préférentielle pour l'une des fractions aqueuse ou organique.

L'étape g) éventuelle de fractionnement d'au moins une partie de la fraction (Ohp1) issue de la zone (Co) de l'étape f), dans une zone (C2), en une fraction (Ohp2) contenant l'oléfine tertiaire et en une fraction (F) contenant la majeure partie des composés légers éventuels présents dans ladite fraction (Ohp1) et éventuellement l'eau résiduelle contenue dans ladite partie de fraction liquide (Ohp1), est habituellement effectuée dans une colonne à distiller fonctionnant sous une pression absolue d'environ 1 à environ 15 bar, le plus souvent d'environ 3 à environ 10 bar, identique ou différente de celle régnant dans la zone de séparation de l'étape f). Pour une unité produisant 0,6 kg/h d'isobutène, cette colonne a habituellement environ 3 à environ 80 plateaux théoriques et le plus souvent environ 5 à environ 50 plateaux théoriques. La température de fond de la colonne dépend en particulier de la pression régnant dans ladite colonne.

L'étape h) éventuelle de fractionnement, dans une zone (C3), d'au moins une partie de la fraction aqueuse (C), contenant la majeure partie de l'alcool initialement présent dans la fraction (A) en une fraction (G) contenant la majeure partie de l'alcool initialement présent dans ladite partie de fraction (C) et en une fraction aqueuse (H) débarrassée de la majeure partie de l'alcool initialement présent dans ladite partie de fraction (C), est habituellement réalisée dans une colonne à distiller (C3) sous une pression absolue d'environ 1 à environ 12 bar, de préférence d'environ 1 à environ 8 bar, identique ou différente de celle régnant dans la zone (L1) d'extraction à l'eau de l'étape e). La température de fond de la colonne dépend en particulier de la pression régnant dans ladite colonne ; elle est habituellement d'environ 50 à environ 300 °C et le plus souvent d'environ 65 à environ 200 °C. La colonne comporte habituellement environ 2 à environ 80 plateaux et le plus souvent environ 3 à environ 60 plateaux théoriques.

Les figures 1 et 2 sont des schémas de principe illustrant chacun une variante préférée de mise en oeuvre de la présente invention. Les traits en pointillés montrent les diverses options possibles.

Dans la première variante illustrée sur la figure 1, la charge hydrocarbonée contenant au moins une oléfine tertiaire est introduite par la ligne 1 dans la zone d'éthérification et de fractionnement (R1). Dans cette zone (R1), on introduit également par la ligne 2 un appoint d'alcool et par la ligne 3a de l'alcool recyclé éventuel. Ladite zone (R1) contient un catalyseur acide d'éthérification. Le produit obtenu par éthérification de la coupe hydrocarboné est scindé dans ladite zone (R1) en une fraction organique hydrocarbonée (O1) qui sort par la ligne 4 et en une fraction organique (E1) contenant l'éther formé qui sort par la ligne 5. Une partie de cette fraction organique (E1) est envoyée par la ligne 5a aux fractions carburant moteur et l'autre partie est envoyée par la ligne 5c dans la zone (R2) de décomposition de l'éther. Par la ligne 6 on récupère un produit (P1) contenant une oléfine tertiaire et de l'alcool que l'on envoie dans la colonne de fractionnement (C1). Le produit contenant de l'alcool sortant de la colonne (C1) par la ligne 3 peut par exemple être en partie envoyé par les lignes 3, 3c puis 3a vers la zone (R1). Une autre partie de ce produit peut être évacuée par les lignes 3 et 3b. Le produit contenant l'oléfine tertiaire est introduit par la ligne 7 dans une zone (L1) d'extraction à l'eau dans laquelle de l'eau est introduite par la ligne 11 et à partir de laquelle on récupère une fraction (D) appauvrie en alcool par la ligne 9 que l'on envoie dans la zone de séparation Co à partir de laquelle on récupère par la ligne 23 une fraction liquide aqueuse (Le) et par la ligne 24 une fraction liquide hydrocarbonée (Ohp1) contenant la majeure partie de l'oléfine tertiaire initialement présente dans la fraction (D). Ladite fraction (Ohp1), contenant ladite oléfine tertiaire, une faible quantité d'eau et éventuellement des composés légers, est envoyée par la ligne 24 dans une zone de fractionnement (C2). Par la ligne 8, on récupère en sortie de la zone (L1) un produit aqueux (2) contenant de l'alcool que l'on introduit dans la zone de fractionnement (C3). A partir de la zone de fractionnement (C2), on récupère par la ligne 28 de l'oléfine tertiaire (Ohp2) ultra pure et par la ligne 27 des produits légers. Lesdits produits légers sont par exemple en partie envoyés à la torche par les lignes 27 et 27a, mais ils peuvent aussi être envoyés dans une zone de craquage catalytique ou bien dans une zone de synthèse d'éther, qui est de préférence la zone (R1), et en contrepartie recyclés à titre de reflux par les lignes 19, 20 et 20b dans la zone de fractionnement (C2). Il est aussi possible et cela est une forme préférée de réalisation d'envoyer au moins une partie de ces produits légers par les lignes 19 et 21 dans une zone de séparation (D1), à partir de laquelle on récupère par la ligne 13 une fraction constituée en majeure partie d'eau, par la ligne 22 une fraction liquide de produits légers que l'on renvoie à titre de reflux dans la colonne (C2) par la ligne 20b, et par la ligne 12 on récupère au moins une partie des produits légers gazeux qui sont par exemple au moins en partie envoyés à la torche, mais qui peuvent aussi être envoyés dans une zone de craquage catalytique ou bien dans une zone, de préférence la zone (R1), de synthèse d'éthers. Il est encore possible de combiner les deux formes de réalisation décrites précédemment. La fraction aqueuse récupérée par la ligne 13 peut être par exemple renvoyée en partie par les lignes 14a, 14, 15 et 11 dans la zone (L1) ou bien récupérée en partie par la ligne 13a. A partir de la zone de fractionnement (C3), on récupère par la ligne 10 de l'alcool qui peut par exemple en partie être envoyé par les lignes 16 et 3a dans la zone (R1) ou bien récupéré par la ligne 10a. A partir de cette zone (C3), on récupère également une fraction aqueuse par la ligne 39 qui peut être envoyée au moins en partie vers une zone de traitement des eaux par la ligne 39a ou recyclée par les lignes 17, 15 et 11 au moins en partie dans la zone (L1). La fraction liquide aqueuse (Le) récupérée par la ligne 23 à partir de la zone de séparation (Co) peut être envoyée au moins en partie vers une zone de traitement des eaux par la ligne 26 ou recyclée par les lignes 25, 14, 15 et 11 au moins en partie dans la zone (L1), en plus d'un appoint éventuel extérieur en eau par la ligne 15b.

La deuxième variante illustrée sur la figure 2 diffère de celle décrite en liaison avec la figure 1 en ce que le produit (E1) sortant de la zone d'éthérification et de (R1) est au moins en partie envoyé par les lignes 5 et 50 dans une zone de purification (C4), l'autre partie, si elle existe, étant envoyée directement par les lignes 5, 5b et 5c dans la zone (R2) de décomposition de l'éther. A partir de la zone de purification (C4) on récupère par la ligne 52 une fraction lourde appauvrie en éther et par la ligne 51 une fraction enrichie en éther qui est envoyée par la ligne 5c dans la zone (R2). Les autres éléments schématisés sur la figure 2 sont similaires à ceux décrits en liaison avec la figure 1. La fraction lourde récupérée par la ligne 52 est par exemple au moins en partie envoyée dans des fractions carburant.

Les exemples suivants illustrent l'invention sans en limiter la portée.

### Exemple 1

On utilise un équipement de type pilote comprenant deux réacteurs tubulaires (R1) et (R2), de volume respectif 20 millilitres et 10 millilitres. Le premier réacteur (R1) contient 6 grammes de résine Amberlyst 15 vendue par la société ROHM ET HAAS et fonctionne sous une pression relative de 10 bar, à une température moyenne de 50 °C. Il est alimenté par une coupe C₄ issue du craquage catalytique contenant 20 % en poids d'isobutène et par du méthanol pur vendu par la société ALDRICH comme un produit ayant une pureté supérieure à 99 % en poids. La quantité de méthanol est ajustée de manière à ce que le rapport molaire méthanol/isobutène introduit dans le réacteur (R1) soit de 1,2. Le réacteur travaille avec une VVH de 0,5 h⁻¹. Dans les conditions choisies la conversion de l'isobutène en MTBE est égale à 97,5 %.

Le deuxième réacteur (R2) contient 3 grammes de catalyseur commercial à base de polysiloxanes greffés par au moins un groupement alkyl sulfonique. On alimente le réacteur R2 par une charge contenant 100 % poids de MTBE, sous une VVH de 15 h⁻¹, la pression relative dans le réacteur est de 7 bar et la température moyenne de 160 °C. Le tableau 1 présente la composition de la charge introduite dans le réacteur R2 de décomposition du MTBE et la composition du produit recueilli à la sortie du réacteur R2.

**Tableau 1**

| | Charge(% poids) | Effluent R2 (% poids) |
|---|---|---|
| MTBE | 100 | 10 |
| Isobutène | | 56,1 |
| Méthanol | | 32,1 |
| DME | | 0,5 |
| Dimères | | 1,1 |
| H2O | | 0,2 |

A l'aide du logiciel commercialisé par la société américaine SIMSCI (SIMulation SCIence INC.) sous la dénomination commerciale Pro II, on calcule les diverses sections de purification.

Une colonne de distillation (Col), fonctionnant sous une pression relative de 7 bar, comportant 10 plateaux théoriques, est utilisée à l'étape b) du procédé de l'invention pour obtenir un produit de fond (E1) et un produit de tête (O1). (On simule ici le fractionnement du produit issu de l'étape a) d'éthérification de l'isobutène contenu dans la coupe (C₄)).

Une colonne de distillation (C1), fonctionnant sous une pression relative de 7 bar, comportant 10 plateaux théoriques, est utilisée à l'étape c) du procédé de l'invention pour obtenir un produit de fond (B) et un produit de tête (A), (On simule ici le fractionnement du produit issu de l'étape c) de décomposition du MTBE)

Une colonne d'extraction par lavage à l'eau (L1), qui est une colonne à plateaux et qui fonctionne à température 30 °C sous une pression relative de 12 bar, est utilisée dans l'étape e) du procédé de l'invention pour obtenir une fraction aqueuse (C) et une fraction organique (D).

Un système d'extraction de l'eau libre entraînée à l'étape e) dans la fraction (D), du type coalesceur (Co), permet d'obtenir une fraction aqueuse (Le) et une fraction organique (Ohp1). Il fonctionne sous une pression relative de 12 bar à une température de 30 °C.

Une colonne de distillation (C2), dernière étape de purification de l'isobutène, fonctionnant sous une pression relative de 7 bar, et-comportant 10 plateaux théoriques, est utilisée à l'étape g) du procédé de l'invention pour obtenir un produit de fond (Ohp2) qui est de l'isobutène purifié et un produit de tête (F) contenant des composés légers.

La colonne (Col) est alimenté par le produit issu du réacteur d'éthérification. Le produit (E1) récupéré en fond de colonne (Col) est utilisé pour alimenter le réacteur (R2). La colonne (C1) est alimentée par l'effluent de (R2). Le produit (A) recueilli en tête de (C1) est envoyé dans la colonne d'extraction (L1) où il est lavé par une quantité d'eau dont le débit volumique est égal au dixième du débit de (A). On recueille une fraction aqueuse (C) contenant la majeure partie du méthanol contenu dans (A), et une fraction hydrocarbonée (D) contenant une petite quantité d'eau libre entraînée. Cette fraction d'eau libre est enfin éliminée après décantation dans le système de décantation (Co) sous forme d'une fraction (Le) et on récupère une fraction organique hydrocarbonée (Ohp1). Enfin, la fraction hydrocarbonée (Ohp1) est traitée dans la colonne (C2) afin de produire en fond de colonne une fraction (Ohp2) qui est de l'isobutène de haute pureté et en tête une fraction légère (F) contenant en particulier le diméthyl éther (DME).

Les bilans matières obtenus sont donnés dans les tableaux 2, 3 et 4 ci-après.

**Tableau 2**

| | Charge R1 (poids) | Effluent R1 (poids) | Tête Col (poids) | Fond Col (poids) |
|---|---|---|---|---|
| Isobutène | 20 | 0,6 | 0,6 | |
| C4 hors isobutène | 80 | 80 | 80 | |
| Méthanol | 13,7 | 2,7 | 2,7 | |
| MTBE | 0 | 30,4 | 0 | 30,4 |
| Total | 113,7 | 113,7 | 83,3 | 30,4 |

**Tableau 3**

| | Effluent R2 (% poids) | Produit B colonne C1 (g/h) | produit A colonne C1 (g/h) | Eau de lavage colonne L1 | fraction C colonne L1 | fraction D colonne L1 |
|---|---|---|---|---|---|---|
| MTBE | 10 | 10 | | | | |
| Isobutène | 56,1 | | 56,1 | | | 56,1 |
| Méthanol | 32,1 | 30,4 | 1,7 | | 1,7 | |
| DME | 0,5 | | 0,5 | | | 0,5 |
| Dimères | 1,1 | 1,1 | | | | |
| H2O | 0,2 | | 0,2 | 10 | 9,2 | 1 |
| Débit (g/h) | 100 | 41,5 | 58,5 | 10 | 10,9 | 57,6 |

**Tableau 4**

| | fraction aqueuse Le extraite par Co | fraction organique Ohp1 sortie de Co | Fraction F colonne C2 | Fraction Ohp2 colonne C2 |
|---|---|---|---|---|
| Isobutène | | 56,1 | 1,72 | 54,38 |
| DME | | 0,5 | 0,49 | 0,01 |
| H2O | 0,99 | 0,01 | 0,01 | |
| Débit (g/h) | 0,99 | 56,61 | 2,22 | 54,39 |
| Pureté de l'isobutène (%) | | | | 99,98 % |

Cet exemple montre que la réalisation du procédé selon l'invention permet d'obtenir à partir d'une coupe C₄ contenant de l'isobutène, un isobutène ayant une pureté de 99,98 %, avec une conversion globale de 82,6 %. La conversion peut être améliorée par l'utilisation de divers recyclages, dont celui de l'alcool non converti et par la purge vers les fractions essence d'une partie de l'éther formé au cours de l'éthérification. Cette conversion peut ainsi dépasser 94 %.

### Exemple 2

On utilise un équipement de type pilote comprenant un réacteur tubulaire R1 et R2 pour réaliser les deux étapes réactionnelles, synthèse et décomposition de l'éther TAME. Les étapes de séparation par distillation et les sections de purification sont calculées à l'aide du logiciel Pro II.

Dans l'unité pilote on réalise, dans un premier temps, la synthèse de l'éther TAME par réaction des isoamylènes avec du méthanol en présence de résine Amberlyst 15 (fabriqué par Rohm & Haas). Pour cela on utilise une coupe C5 de type FCC contenant 22 % poids d'isoamylènes (méthyl-2 butène-1 et méthyl-2 butène-2) additionné de méthanol (origine Aldrich, pureté > 99 % poids), de façon à disposer d'une charge de stoechiométrie méthanol/isoamylènes de 1. L'unité équipé du réacteur R1 est opéré à 10 bar (pression relative) et 60 °C, sous une VVH de 0,5 h-1. Au cours de cette étape, la conversion des isoamylènes en TAME s'élève à 67 %.

Ensuite, l'unité pilote est opérée dans des conditions opératoires différentes pour réaliser la réaction inverse de décomposition du TAME. Le réacteur R2 contient du catalyseur Deloxan ASP (fabriqué par Degussa). L'unité est opéré sous une pression relative de 7 bar, à une température moyenne de 140 °C. On alimente le réacteur R2 par une charge contenant 100 % poids de TAME, sous une VVH de 6 h-1. Le produit recueilli à la sortie du réacteur R2 possède la composition donnée dans le tableau 5 :

**Tableau 5 :**

| Section réactionnelle de décomposition du TAME | | |
|---|---|---|
| | Charge (% poids) | Effluent R2 (% poids) |
| TAME | 100 | 15 |
| Isoamylènes | | 58,2 |
| Méthanol | | 26,45 |
| DME | | 0,25 |
| Dimères | | 0,1 |

A l'aide du logiciel Pro II, les divers sections de purification sont calculés comme dans l'exemple précédent.

**Tableau 6 :**

| Bilan matière relatif à la synthèse du TAME | | | | |
|---|---|---|---|---|
| | Charge R1 (poids) | Effluent R1 (poids) | Tête C1 (poids) | Fond C1 (poids) |
| Isoamylènes | 22 | 7,26 | 7,26 | |
| C5 hors isoamylènes∗ | 78 | 78 | 78 | |
| Méthanol | 10,1 | 3,36 | 3,36 | |
| TAME | 0 | 21,48 | | 21,48 |
| Total | 110,1 | 110,1 | 88,62 | 21,48 |

| | | | | |
|---|---|---|---|---|
| ∗ Est inclu ici le méthyl-3 butène-1, oléfine classée dans les "non-éthérifiable". | | | | |

**Tableau 7 :**

| Bilan matière relatif à la décomposition du TAME et à la production d'isoamylènes de haute pureté. | | | | | | |
|---|---|---|---|---|---|---|
| | Effluent R2 (% poids) | Fond C2 (g/h) | Tête C2 ou produit A | Eau de lavage | fraction C | fraction D |
| TAME | 15 | 15 | | | | |
| Isoamylènes | 58,2 | | 58,2 | | | 58,2 |
| Méthanol | 26,45 | 24,45 | 2 | | 2 | |
| DME | 0,25 | | 0,25 | | | 0,25 |
| Dimères | 0,1 | 0,1 | | | | |
| H2O | | | | 12 | 11,5 | 0,5 |
| Débit (g/h) | 100 | 39,55 | 60,45 | 12 | 13,5 | 58,95 |

L'intégration des deux procédés (synthèse du TAME puis décomposition du TAME) permet d'extraire les isoamylènes (méthyl-2 butène-1 et méthyl-2 butène-2) d'une coupe C5 avec un rendement minimum de 56 %. (rendement pouvant être amélioré par le biais de recyclage de l'éther non converti) et avec une pureté supérieur à 98 %.

## Revendications

1. Procédé de production d'oléfine tertiaire pure et/ou d'éther alkylique tertiaire à partir de coupe hydrocarbonée contenant au moins une oléfine tertiaire éthérifiable, le procédé comprenant :
a) une étape de formation d'au moins un éther alkylique tertiaire par mise en contact dans une zone réactionnelle (R1), contenant un catalyseur d'éthérification, d'au moins une coupe hydrocarbonée contenant au moins une oléfine tertiaire éthérifiable avec au moins un alcool,
b) une étape de séparation de la majeure partie du produit issu de l'étape a) en une fraction organique (O1) appauvrie en éther alkylique tertiaire et en une fraction organique (E1) enrichie en éther alkylique tertiaire formé au cours de l'étape a),
c) une étape de décomposition d'au moins une partie de l'éther alkylique tertiaire contenu dans la fraction organique (E1) de l'étape b), dans une zone réactionnelle (R2) contenant un catalyseur de décomposition dudit éther, en un produit (P1) contenant au moins un alcool et au moins une oléfine tertiaire,
d) une étape de fractionnement d'au moins une partie du produit (P1), dans une zone de fractionnement (C1) permettant d'obtenir d'une part une fraction (A) contenant la majeure partie de l'oléfine tertiaire et éventuellement une fraction mineure d'alcool et des composés légers éventuels contenus initialement dans ladite partie de produit (P1), et d'autre part une fraction (B) contenant la majeure partie de l'alcool formé dans l'étape c) et éventuellement de l'éther non décomposé dans l'étape c),
e) une étape de purification d'au moins une partie de la fraction (A) dans laquelle ladite partie est envoyée dans une zone (L1) d'extraction par lavage à l'eau à partir de laquelle on obtient une fraction aqueuse (C) contenant la majeure partie de l'alcool initialement présent dans ladite partie et une fraction(D) contenant la majeure partie de l'oléfine tertiaire initialement présente dans ladite partie, ladite fraction (D) contenant ladite oléfine tertiaire, de l'eau, éventuellement des composés légers et étant sensiblement exempte d'alcool,
ledit procédé étant **caractérisé en ce qu**'il comporte une étape f) dans laquelle au moins une partie de la fraction (D) est envoyée dans une zone de séparation (Co) à partir de laquelle on récupère une fraction liquide aqueuse (Le) et une fraction organique liquide (Ohp1) contenant la majeure partie de l'oléfine tertiaire initialement présente dans ladite partie de fraction (D), ladite fraction (Ohp1) contenant ladite oléfine tertiaire, une faible quantité d'eau et éventuellement des composés légers étant ultérieurement purifiée par fractionnement.

2. Procédé selon la revendication 1 dans lequel une partie de la fraction organique (E1) contenant de l'éther alkylique tertiaire est envoyée dans des fractions carburant moteur et 1 autre partie est envoyée à l'étape c) de décomposition de l'éther alkylique tertiaire.

3. Procédé selon la revendication 1 ou 2 comprenant une étape bl) dans laquelle au moins une partie de la fraction organique (E1) issue de l'étape b) contenant de l'éther alkylique tertiaire est envoyée dans une zone de purification (C4), à partir de laquelle on obtient une fraction lourde (L1) appauvrie en éther alkylique tertiaire et une fraction plus légère (E2) enrichie en éther alkylique tertiaire que l'on envoie à l'étape c) de décomposition dudit éther.

4. Procédé selon l'une des revendications 1 à 3 dans lequel au moins une partie de la fraction liquide (Ohp1) récupérée à l'étape f) est envoyée dans une étape g) dans une zone de fractionnement (C2) dans laquelle ladite partie de la fraction liquide (Ohp1) est fractionnée en une fraction (Ohp2) contenant l'oléfine tertiaire et en une fraction (F) contenant la majeure partie des composés légers éventuels et éventuellement une faible quantité d'eau résiduelle.

5. Procédé selon la revendication 4 dans lequel la zone de fractionnement de l'étape g) comporte au moins un moyen permettant de récupérer à partir de la fraction (F) une fraction légère sensiblement anhydre.

6. Procédé selon la revendication 5 dans lequel ledit moyen comprend un ballon séparateur muni d'au moins un moyen permettant la décantation et le soutirage d'une fraction aqueuse.

7. Procédé selon la revendication 6 dans lequel la fraction aqueuse obtenue à l'étape g) est au moins en partie recyclée à l'étape e) dans la zone (L1) d'extraction par lavage à l'eau.

8. Procédé selon l'une des revendications 4 à 7 dans lequel au moins une partie de la fraction (F) ou de la fraction légère sensiblement anhydre obtenue à l'étape g) à partir de ladite fraction (F) est au moins en partie envoyée dans une zone de craquage catalytique.

9. Procédé selon l'une des revendications 4 à 8 dans lequel au moins une partie de la fraction (F) ou de la fraction légère sensiblement anhydre obtenue à l'étape g) à partir de ladite fraction (F) est au moins en partie envoyée dans la zone (R1) de synthèse d'éther de l'étape a).

10. Procédé selon l'une des revendications 1 à 9 comprenant une étape h) dans laquelle au moins une partie de la. fraction (C) issue de l'étape e) est envoyée dans une zone de fractionnement (C3) à partir de laquelle on récupère une fraction (G) contenant la majeure partie de l'alcool initialement présent dans ladite partie et une fraction aqueuse (H) débarrassée de la majeure partie de l'alcool initialement présent dans ladite partie.

11. Procédé selon la revendication 10 dans lequel au moins une partie de la fraction (G) obtenue à l'étape h) contenant de l'alcool est envoyée dans la zone (R1) synthèse d'éther de l'étape a).

12. Procédé selon l'une des revendications 10 ou 11 dans lequel au moins une partie de la fraction aqueuse (H) obtenue à l'étape h) est envoyée au moins en partie dans une zone de traitement des eaux.

13. Procédé selon l'une des revendications 10 à 12 dans lequel au moins une partie de la fraction aqueuse (H) obtenue à l'étape h) est au moins en partie recyclée à l'étape e) dans la zone (L1) d'extraction par lavage à l'eau.

14. Procédé selon l'une des revendications 1 à 13 dans lequel au moins une partie de la fraction (B) obtenue à l'étape d) contenant de l'alcool est envoyée dans la zone (R1) de synthèse d'éther de l'étape a).

15. Procédé selon l'une des revendications 1 à 14 dans lequel au moins une partie de la fraction aqueuse (Le) obtenue à l'étape f) est au moins en partie recyclée à l'étape e) dans la zone (L1) d'extraction par lavage à l'eau.

16. Procédé selon l'une des revendications 1 à 15 dans lequel à l'étape e) de purification d'au moins une partie de la fraction (A) on introduit dans la zone (L1) d'extraction par lavage à l'eau une quantité d'eau telle que le rapport volumique entre le volume de ladite quantité d'eau introduite dans ladite zone d'extraction et celui de ladite partie de fraction (A) introduite dans ladite zone d'extraction (Vₑₐᵤ/V_{A}) est de 0,005 à 20.

17. Procédé selon l'une des revendications 1 à 16 dans lequel la zone (L1) d'extraction par lavage à l'eau de l'étape e) comporte au moins un moyen d'introduction d'eau d'appoint.

18. Procédé selon l'une des revendications 1 à 17 dans lequel le catalyseur de synthèse d'éther alkylique tertiaire est choisi dans le groupe formé par les résines acides organiques et les résines acides minérales.

19. Procédé selon l'une des revendications 1 à 18 dans lequel le catalyseur de décomposition de l'éther alkylique tertiaire est choisi dans le groupe formé par les résines acides organiques et les résines acides minérales.

20. Procédé selon l'une des revendications 1 à 19 dans lequel le catalyseur de décomposition de l'éther alkylique tertiaire est choisi dans le groupe formé par les solides minéraux greffés comportant au moins un groupe organique de type alkyl-sulfonique, aryl-sulfonique ou alkylaryl-sulfonique.

21. Procédé selon l'une des revendications 1 à 20 dans lequel le catalyseur de décomposition de l'éther alkylique tertiaire est choisi dans le groupe formé par les polysiloxanes greffés par au moins un groupe alkyl-sulfonique.

## Patentansprüche

1. Verfahren zur Herstellung reinen tertiären Olefins, und/oder tertiären Alkylethers ausgehend von einem kohlenwasserstoffhaltigen Schnitt, der wenigstens ein tertiäres etherifizierbares Olefin enthält, wobei das Verfahren umfasst:
a. eine Stufe der Bildung wenigstens eines tertiären Alkylethers durch in Kontakt bringen wenigstens eines kohlenwasserstoffhaltigen Schnittes, der wenigstens ein tertiäres etherifizierbares Olefin enthält, mit wenigstens einem Alkohol in einer Reaktionszone (R1), die einen Etherifzierungskatalysator enthält,
b. eine Stufe der Trennung des größeren Teils des aus der Stufe a) stammenden Produkts in eine organische, an tertiärem Alkylether verarmte Fraktion (O1) und in eine organische Fraktion (E1), die an dem während der Stufe a) gebildeten tertiären Alkylether angereichert ist,
c. eine Stufe der Zersetzung wenigstens eines Teils des tertiären Alkylethers, der in der organischen Fraktion (E1) der Stufe b) enthalten ist, in einer Reaktionszone (R2), die einen Zersetzungskatalysator dieses Ethers enthält, zu einem Produkt (P1), welches wenigstens einen Alkohol und wenigstens ein tertiäres Olefin enthält,
d. eine Stufe der Fraktionierung wenigstens eines Teils des Produkts (P1) in einer Fraktionierungszone (C1), die es ermöglicht, einerseits eine Fraktion (A) zu erhalten, die den größeren Teil des tertiären Olefins enthält und gegebenenfalls eine kleinere Fraktion Alkohol und eventuelle, anfänglich in diesem Teil des Produkts (P1) enthaltene leichte Verbindungen, und andererseits eine Fraktion (B), die den größeren Teil des in der Stufe c) gebildeten Alkohols und gegebenenfalls in der Stufe c) nicht zersetzten Ether enthält,
e. eine Stufe zur Reinigung wenigstens eines Teils der Fraktion (A), in der dieser Teil in eine Zone (L1) der Extraktion durch Waschen mit Wasser geschickt wird, ausgehend von der man eine wässrige Fraktion (C) erhält, die den größeren Teil des anfänglich in diesem Teil vorliegenden Alkohols und eine Fraktion (D) enthält, die den größeren Teil des anfänglich in diesem Teil vorliegenden tertiären Olefins enthält, wobei diese Fraktion (D) dieses tertiäre Olefin, Wasser und gegebenenfalls leichte Verbindungen enthält und im wesentlichen frei von Alkohol ist,
wobei das Verfahren **dadurch gekennzeichnet** ist, dass es eine Stufe f) umfasst, in der wenigstens ein Teil der Fraktion (D) in eine Trennzone (Co) geschickt wird, ausgehend von der man eine wässrige, flüssige Fraktion (Le) und eine organische, flüssige Fraktion (Ohp1) gewinnt, die den größeren Teil des anfänglich in der Fraktion (D) vorliegenden Alkohols enthält, wobei diese Fraktion (Ohp1) dieses tertiäre Olefin, eine geringe Menge Wasser und gegebenenfalls leichte Verbindungen enthält und später durch Fraktionierung gereinigt wird.

2. Verfahren nach Anspruch 1, in dem wenigstens ein Teil der organischen, tertiären Alkylether enthaltenden Fraktion (E1) in die Motortreibstofffraktionen geschickt wird und ein anderer Teil zur Zersetzungsstufe f) von tertiärem A1kylether geschickt wird.

3. Verfahren nach einem der Ansprüche 1 oder 2, welches eine Stufe b1) umfasst, in der wenigstens ein Teil der organischen aus der Stufe b) stammenden, tertiären Alkylether enthaltenden Fraktion (E1) in eine Reinigungszone (C4) geschickt wird, ausgehend von der man eine schwere Fraktion (L1) erhält, die an tertiärem Alkylether verarmt ist und eine leichtere Fraktion (E2), die an tertiärem Alkylether angereichert ist und die man zur Stufe c) der Zersetzung dieses Ethers schickt.

4. Verfahren nach einem der Ansprüche 1 bis 3, in dem wenigstens ein Teil der in Stufe f) gewonnenen flüssigen Fraktion (Ohp1) in einer Stufe g) in eine Fraktionierungszone (C2) geschickt wird, in der dieser Teil der flüssigen Fraktion (Ohp1) in eine das tertiäre Olefin enthaltende Fraktion (Ohp2) und in eine Fraktion (F2) fraktioniert wird, die den größeren Teil der eventuellen leichten Verbindungen und gegebenenfalls eine geringe Menge restlichen Wassers enthält.

5. Verfahren nach Anspruch 4, in dem die Fraktionierungszone der Stufe g) wenigstens ein Mittel umfasst, das es ermöglicht, ausgehend von der Fraktion (F) eine leichte, im wesentlichen wasserfreie Fraktion zu gewinnen.

6. Verfahren nach Anspruch 5, in dem dieses Mittel ein Trennbehälter ist, der mit wenigstens einem Mittel ausgerüstet ist, das die Dekantierung und das Abziehen der wässrigen Fraktion ermöglicht.

7. Verfahren nach Anspruch 6, in dem die wässrige, in Stufe g) erhaltene Fraktion wenigstens zum Teil in Stufe e) in die Extraktionszone (L1) durch Waschen mit Wasser rezykliert wird.

8. Verfahren nach einem der Ansprüche 4 bis 7, in dem wenigstens ein Teil der Fraktion (F) oder der leichten, im wesentlichen wasserfreien, Fraktion, die in Stufe g) ausgehend von der Fraktion (F) erhalten wurde, wenigstens zum Teil in eine Zone katalytischen Crackens geschickt wird.

9. Verfahren nach einem der Ansprüche 4 bis 8, in dem wenigstens ein Teil der Fraktion (F) oder der leichten, im wesentlichen wasserfreien, Fraktion, die in Stufe g) ausgehend von der Fraktion (F) erhalten wurde, wenigstens zum Teil in die Zone (R2) der Ethersynthese der Stufe a) geschickt wird.

10. Verfahren nach einem der Ansprüche 1 bis 9, das eine Stufe h) umfasst, in der wenigstens ein Teil der aus der Stufe e) stammenden Fraktion (C) in eine Fraktionierungszone (C3) geschickt wird, ausgehend von der man eine Fraktion (G) gewinnt, die den größeren Teil des anfänglich in diesem Teil vorliegenden Alkohols und eine wässrige Fraktion (H) enthält, die vom größeren Teil des anfänglich in diesem Teil vorliegenden Alkohols befreit ist.

11. Verfahren nach Anspruch 10, in dem wenigstens ein Teil der in Stufe h) erhaltenen Fraktion (G), die Alkohol enthält, in die Zone (R1) der Ethersynthese der Stufe a) geschickt wird.

12. Verfahren nach einem der Ansprüche 10 oder 11, in dem wenigstens ein Teil der bei Stufe h) erhaltenen wässrigen Fraktion (H) wenigsten zum Teil in eine Wasserbehandlungszone geschickt wird.

13. Verfahren nach einem der Ansprüche 10 bis 12, in dem wenigstens ein Teil der bei Stufe h) erhaltenen wässrigen Fraktion (H) wenigstens zum Teil zur Stufe e) in die Extraktionszone (L1) durch Waschen mit Wasser geschickt wird.

14. Verfahren einem der Ansprüche 1 bis 13, in dem wenigstens ein Teil der in Stufe d) erhaltenen Fraktion (B), die Alkohol enthält, in die Zone (R1) der Ethersynthese der Stufe a) geschickt wird.

15. Verfahren nach einem der Ansprüche 1 bis 14, in dem wenigstens ein Teil der wässrigen, in Stufe f) erhaltenen Fraktion (Le) wenigstens zum Teil zur Stufe f) in die Extraktionszone (L1) durch Waschen mit Wasser rezykliert wird.

16. Verfahren nach einem der Ansprüche 1 bis 15, in dem man bei Stufe e) der Reinigung wenigstens eines Teils der Fraktion (A) eine Wassermenge in die Extraktionszone (L1) durch Waschen mit Wasser derart einführt, dass das Volumenverhältnis zwischen dem Volumen dieser in diese Extraktionszone eingeführten Wassermenge und jenem des in diese Extraktionszone eingeführten Teils der Fraktion (A) (V_{wasser}/V_{A}) 0,005 bis 20 ist.

17. Verfahren nach einem der Ansprüche 1 bis 16, in dem die Extraktionszone (L1) durch Waschen mit Wasser der Stufe e) wenigstens ein Mittel zum Einführen zusätzlichen Wassers enthält.

18. Verfahren nach einem der Ansprüche 1 bis 17, in dem der Katalysator der Synthese tertiären Alkylethers in der durch die sauren organischen Harze und die sauren mineralischen Harze gebildeten Gruppe gewählt ist.

19. Verfahren nach einem der Ansprüche 1 bis 18, in dem der Katalysator der Zersetzung tertiären Alkylethers in der durch die sauren organischen Harze und die sauren mineralischen Harze gebildeten Gruppe gewählt ist.

20. Verfahren nach einem der Ansprüche 1 bis 19, in dem der Katalysator der Zersetzung tertiären Alkylethers in der durch die gepfropften mineralischen Feststoffe, die wenigstens eine organische Gruppe vom Typ Alkylsulfon, Arylsulfon oder Alkyl-Arylsulfon aufweisen, gebildeten Gruppe gewählt ist.

21. Verfahren nach einem der Ansprüche 1 bis 20, in dem der Katalysator der Zersetzung tertiären Alkylethers in der durch wenigstens eine Alkylsulfongruppe gepfropften Polysiloxane gebildeten Gruppe gewählt ist.

## Claims

1. A process for the production of a pure tertiary olefin and/or a tertiary alkyl ether from a hydrocarbon cut containing at least one etherifiable tertiary olefin, the process comprising:
a) a step for forming at least one tertiary alkyl ether by bringing at least one hydrocarbon cut containing at least one etherifiable tertiary olefin into contact with at least one alcohol in a reaction zone (R1) containing an etherification catalyst;
b) a step for separating the major portion of the product from step a) into an organic fraction (O1) which is depleted in tertiary alkyl ether and an organic fraction (E1) which is enriched in the tertiary alkyl ether formed during step a);
c) a step for decomposing at least a portion of the tertiary alkyl ether contained in the organic fraction (E1) from step b), in a reaction zone (R2) containing a catalyst for decomposing said ether, into a product (P1) containing at least one alcohol and at least one tertiary olefin;
d) a step for fractionating at least a portion of product (P1) in a fractionation zone (C1) to obtain a fraction (A) containing the major portion of the tertiary olefin and possibly a minor fraction of the alcohol and possibly light compounds, initially contained in said portion of product (P1), and a fraction (B) containing the major portion of the alcohol formed in step c) and possibly ether which has not decomposed in step c);
e) a step for purifying at least a portion of fraction (A) in which said portion is sent to a water washing extraction zone (L1) from which an aqueous fraction (C) is obtained containing the major portion of the alcohol initially present in said portion and a fraction (D) is obtained containing the major portion of the tertiary olefin initially present in said portion, said fraction (D) containing said tertiary olefin, water, possibly light compounds and being substantially free of alcohol;
said process being **characterized in that** it comprises a step f) in which at least a portion of fraction (D) is sent to a separation zone (Co) from which an aqueous liquid fraction (Le) and an organic liquid fraction (Ohpl) containing the major portion of the tertiary olefin initially present in said portion of fraction (D) are recovered, said fraction (Ohpl) containing said tertiary olefin, a small quantity of water and possibly light compounds is later purified by fractionating.

2. A process according to claim 1, in which a portion of the organic fraction (E1) containing tertiary alkyl ether is sent for motor fuel pool and the other portion is sent to tertiary alkyl ether decomposition step c).

3. A process according to claim 1 or claim 2, comprising a step b1) in which at least a portion of the organic fraction (E1) from step b) containing the tertiary alkyl ether is sent to a purification zone (C4) from which a heavy fraction (L1) which is depleted in tertiary alkyl ether is obtained, also a lighter fraction (E2) which is enriched in tertiary alkyl ether is obtained which is sent to step c) for decomposing said ether.

4. A process according to any one of claims 1 to 3, in which at least a portion of the liquid fraction (Ohp1) recovered from step f) is sent in a step g) to a fractionation zone (C2) in which said portion of the liquid fraction (Ohp1) is fractionated into a fraction (Ohp2) containing the tertiary olefin and a fraction (F) containing the major portion of any light compounds and possibly a small quantity of residual water.

5. A process according to claim 4, in which the fractionation zone of step g) comprises at least one means for recovering a substantially anhydrous light fraction from fraction (F).

6. A process according to claim 5, in which said means comprises a separator drum provided with at least one means for decanting and extracting an aqueous fraction.

7. A process according to claim 6, in which the aqueous fraction obtained in step g) is recycled at least in part to step e) in water washing extraction zone (L1).

8. A process according to any one of claims 4 to 7, in which at least a portion of fraction (F) or the substantially anhydrous light fraction obtained in step g) from said fraction (F) is sent at least in part to a catalytic cracking zone.

9. A process according to any one of claims 4 to 8, in which at least a portion of fraction (F) or the substantially anhydrous light fraction obtained in step g) from said fraction (F) is sent at least in part to the ether synthesis zone (R1) of step a).

10. A process according to any one of claims 1 to 9, comprising a step h) in which at least a portion of the fraction (C) from step e) is sent to a fractionation zone (C3) from which a fraction (G) containing the major portion of the alcohol initially present in said portion and an aqueous fraction (H) which is free of the major portion of the alcohol initially present in said portion are recovered.

11. A process according to claim 10, in which at least a portion of fraction (G) obtained from step h) containing alcohol is sent to the ether synthesis zone (R1) of step a).

12. A process according to claim 10 or claim 11, in which at least a portion of the aqueous fraction (H) obtained from step h) is sent at least in part to a water treatment zone.

13. A process according to any one of claims 10 to 12, in which at least a portion of fraction (H) obtained from step h) is recycled at least in part to step e) of water washing extraction zone (L1).

14. A process according to any one of claims 1 to 13, in which at least a portion of fraction (B) obtained from step d) containing alcohol is sent to the ether synthesis zone (R1) of step a).

15. A process according to any one of claims 1 to 14, in which at least a portion of the aqueous fraction (Le) obtained from step f) is recycled at least in part to step e) in water washing extraction zone (L1).

16. A process according to any one of claims 1 to 15, in which in step e) for purifying at least a portion of fraction (A), a quantity of water is introduced into water washing extraction zone (L1) such that the volume ratio between the volume of said quantity of water introduced into said extraction zone and that of said portion of fraction (A) introduced into said extraction zone (V_{water}/V_{A}) is 0.005 to 20.

17. A process according to any one of claims 1 to 16, in which the water washing extraction zone (L1) of step e) comprises at least one means for introducing a water makeup.

18. A process according to any one of claims 1 to 17, in which the tertiary alkyl ether synthesis catalyst is selected from the group formed by organic acid resins and mineral acid resins.

19. A process according to any one of claims 1 to 18, in which the tertiary alkyl ether decomposition catalyst is selected from the group formed by organic acid resins and mineral acid resins.

20. A process according to any one of claims 1 to 19, in which the tertiary alkyl ether decomposition catalyst is selected from the group formed by grafted mineral solids comprising at least one organic alkylsulphonic, arylsulphonic or alkylarylsulphonic type group.

21. A process according to any one of claims 1 to 20, in which the tertiary alkyl ether decomposition catalyst is selected from the group formed by polysiloxanes grafted with at least one alkylsulphonic group.
